# EUROPEAN PATENT APPLICATION

(11) **EP 2 319 827 A1**
(43) Date of publication of application: **11.05.2011**
(21) Application number: 09175447.3
(22) Date of filing: 09.11.2009
(51) Int. Cl.: C07C 231/24, C07C 233/18

(54) **Process for the production of polymorph form I of agomelatine**

(71) Applicant: Ratiopharm GmbH, 89079 Ulm (DE)
(72) Inventor: Fischer, Dirk, 89073 Ulm (DE)
(74) Representative: Best, Michael

(57) **Abstract**

The invention provides a process for the preparation of the polymorph from I of agomelatine, characterized by the following crystal data:

C₁₅H₁₇NO₂

Mr = 243.30
Orthorhombic
Pca2₁
a = 31.501 (4) A
b = 9.5280 (10) A
c = 17.906 (2) A
Z=16

the process comprising the process steps of:
a) dissolving agomelatine in a water-miscible solvent to obtain a solution,
b) adding the solution to water having a temperature of 30°C or below, whereby crystals of the polymorph form I of agomelatine are formed,
c) isolating the crystals of the polymorph form I of agomelatine and
d) optionally drying the isolated crystals of the polymorph form I of agomelatine.

## Description

The present invention relates to a process for preparing polymorph form I of agomelatine and a process for preparing medicaments with the polymorph form I of agomelatine, which includes the process for the preparation of the polymorph form I of agomelatine. The present invention also relates to the polymorph form I of agomelatine, which is obtainable by the new process and medicaments containing this polymorph form I of agomelatine.

Melatonin is a neurohormone that is physiologically synthesized principally in the pineal gland and is involved in the regulation of many physiological and pathophysiological processes such as sleep, seasonal disorders, depression and ageing. Melatonin exhibits its physiological actions by activating G-protein-coupled melatonin receptors MT1 and MT2.

Due to its involvement in several physiological and pathophysiological processes melatonin was considered as a possible therapeutically usable substance. However, its therapeutical usage is limited by the fast metabolic degradation. The plasma half-life of melatonin is only about 15 minutes, which strongly restricts its therapeutical usability.

Research efforts have been made for structurally modified melatonin analogues which maintain the pharmacological activity of melatonin but which have improved pharmacokinetic properties. As a result of this research the bioisosterically modified melatonin analogue agomelatine attracted attention as a suitable therapeutic substance. Agomelatine has the chemical name N-[2-(7-methoxy-1-naphthyl)ethyl]acetamide and has the following chemical structure:

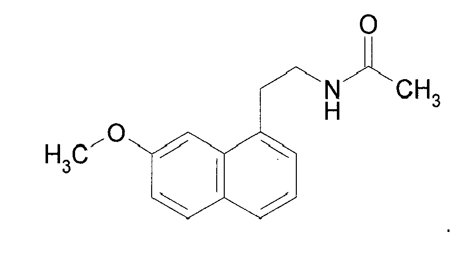

Agomelatine, its preparation and use have first been described in EP-A 0 447 285. It is a pharmacological agent with a dual mechanism of action. Similar to melatonin, agomelatine is an agonist of the melatonergic MT1 and MT2 receptors and, additionally, an antagonist of the 5-HT2c receptors. In the following agomelatine has been extensively investigated.

S. Yous et al. in J. Med. Chem. 1992, 35, 1484-1486 describe a process for the preparation of agomelatine, wherein agomelatine is obtained from a biphasic medium of water and chloroform. Bernard Tinant and Jean-Paul Declercq in Acta Cryst. (1994), C50, 907-910 disclose a full crystallographic investigation of the agomelatine produced by Yous et al. The polymorph form of the product obtained by Yous et al. and analyzed by Tinant and Declercq is designated as the polymorph form I of agomelatine. Tinant and Declercq provide the full crystal data of this polymorph form, and regarding the identification of the polymorph form I of agomelatine, it is explicitly referred to Bernard Tinant and Jean-Paul Declercq in Acta Cryst. (1994), C50, 907-910. The most important crystal data is as follows:

C₁₅H₁₇NO₂

M r = 243. 30
Orthorhombic
Pca2₁
a = 31.501 (4) A
b = 9.5280 (10) Å
c = 17.906 (2) A.

The density is D = 1.203 Mg • m⁻³, and the number of molecules in one cell is Z = 16.

Tinant and Declercq also disclose the fractional atomic coordinates and equivalent isotropic displacement parameters and selected geometric parameters as well as the hydrogen bonding geometry of this polymorph form. This data is included herein by reference.

EP 0 447 285 in example 1 also discloses a process for the preparation of agomelatine. In this process agomelatine is obtained by recrystallization from isopropyl ether. No information on the polymorph form can be found in EP 0 447 285.

Later publications consider that the agomelatine obtained according to document D1 is the same polymorphic form obtained by Yous et al. (e.g. US 2005/0182276) but no experimental data is provided. The EMEA Guidelines (EMEA ICH Topic Q 3 C (R3) Impurities: Residual Solvents, March 1998, CPMP/ICH/283/95; if in this specification reference is made to the EMEA guidelines, these guidelines are meant) list isopropyl ether as a solvent for which no adequate toxicological data is available.

The known processes for obtaining the polymorphic form I of agomelatine are considered as providing the polymorphic form I of agomelatine not in a sufficiently reproducible and well-defined manner and with only poor filtrability (US 2005/0182276) . Furthermore, the known methods of preparation of polymorph form I of agomelatine use solvents which can be problematic and which should be avoided in medicaments. In particular, chloroform, that has been used by Yous et al. for the preparation of agomelatine of polymorphic form I, is a class II solvent with a concentration limit of only 60 ppm in pharmaceutical products. It is very difficult to remove the hazardous solvent to the required extent from the agomelatine.

Several processes have been developed to prepare agomelatine crystals using less problematic solvents and to overcome the problems of the prior art processes. However, none of these processes produced the polymorphic form I of agomelatine, but different new polymorphic forms of agomelatine were obtained. In particular, an attempt to dissolve agomelatine in a water-soluble solvent that is essentially acceptable in pharmaceutical compositions, such as ethanol, and crystallizing the agomelatine by adding water to the solution resulted in a new polymorphic form which was designated polymorphic form II of agomelatine. This polymorphic form II has a monoclinic crystal lattice and not an orthorhombic crystal lattice and a different space group (namely P2₁/N). Form II has eight molecules in the unit cell and not 16 as the polymorphic form I of agomelatine and has lattice parameters A = 20.0903 Å, B = 9.3194 Å and C = 15.4796 Å. This polymorphic form (polymorphic form II) of agomelatine is described e.g. in EP-A 1 564 202 and US 2005/0182276. In the process developed in these documents the agomelatine is prepared in ethanol, and water is added to the hot ethanol to precipitate the agomelatine.

Other processes are also known for the preparation of crystal forms of agomelatine, but those processes lead to other polymorphic forms of agomelatine. In particular, US 2009/0069434 discloses a process which leads to crystalline form VI of agomelatine, US 2006/0270876 discloses a process which leads to a polymorphic form III of agomelatine, US 2006/0270877 discloses a process which leads to the polymorphic form V of agomelatine, and US 2006/0270875 discloses a process which leads to the polymorphic form IV of agomelatine.

A polymorph form of agomelatine for use in a medicament should have excellent properties, such as crystallinity, polymorphic stability, chemical stability and processability to pharmaceutical compositions.

Therefore, there exists a need in the art for a process for reliably and reproducibly producing the polymorphic form I of agomelatine with good filtrability, in particular for a process in which solvents are used which are better pharmaceutically acceptable than the solvents used in the prior art processes for obtaining the polymorphic form I of agomelatine. Furthermore, the polymorph form I of agomelatine should be provided in a very high chemical and polymorph purity.

Unexpectedly it was found that the polymorphic form I of agomelatine can be reliably obtained by an easy process in excellent reproducibility using a wide variety of different solvents, if solvents are used which are miscible with water and certain process conditions are maintained. The new process allows in particular the use of pharmaceutically acceptable solvents such as ethanol for the preparation of agomelatine of polymorphic form I. The obtained polymorph form I of agomelatine has a chemical purity of preferably 98% or more, more preferably 99% or more, in particular 99.5% or more, such as 99.7% or more. The obtained polymorph form I of agomelatine has a polymorph purity of preferably 98% or more, more preferred 99% or more, in particular 99.5% or more, such as 99.7% or more (all %-values in this context are % by weight).

The present invention provides a process for the preparation of the polymorph from I of agomelatine, characterized by the following crystal data:

C₁₅H₁₇NO₂

Mr = 243.30
Orthorhombic
Pca2₁
a = 31.501 (4) Å
b = 9.5280 (10) Å
c = 17.906 (2) Å
Z=16
the process comprising the process steps of:
a) dissolving agomelatine in a water-miscible solvent to obtain a solution,
b) adding the solution to water having a temperature of 30°C or below, whereby crystals of the polymorph form I of agomelatine are formed,
c) isolating the crystals of the polymorph form I of agomelatine and
d) optionally drying the isolated crystals of the polymorph form I of agomelatine.

The present invention also provides a process for the preparation of a medicament containing as active ingredient the polymorph form I of agomelatine as defined in claim 1, comprising the following steps:
i) carrying out a process as defined in any of claims 1 to 7 in order to obtain the polymorph form I of agomelatine as defined in claim 1 and
ii) mixing the polymorph form I of agomelatine obtained in step i) with one or more pharmaceutically acceptable excipients.

The agomelatine obtained by the process of the present invention is of the known polymorphic form I. However, since water-soluble solvents have been used for the preparation of the polymorphic form I of agomelatine, which were never used in the prior art before, the polymorphic form I of agomelatine obtained by the process of the invention can be distinguished from the previously known polymorphic form I of agomelatine, since the remaining solvent molecules in the product are different from the solvent molecules in the prior art products. The polymorphic form I of agomelatine obtainable by the process of the present invention usually contains 10 to 500 ppm, preferably 20 to 300 ppm, more preferably 50 to 200 ppm of the water-miscible solvent, preferably of methanol or ethanol. The polymorph form I of agomelatine obtained by the process of the present invention is distinguishable and thus novel over the product obtained by the prior art processes. The present invention also relates to those novel products and to pharmaceutical compositions containing the novel compounds.

The agomelatine of polymorph form I obtained by the process of the present invention has the required excellent properties regarding crystallinity, polymorph stability, chemical stability, filtrability and processability to pharmaceutical compositions and a high chemical and polymorph purity, as indicated above.

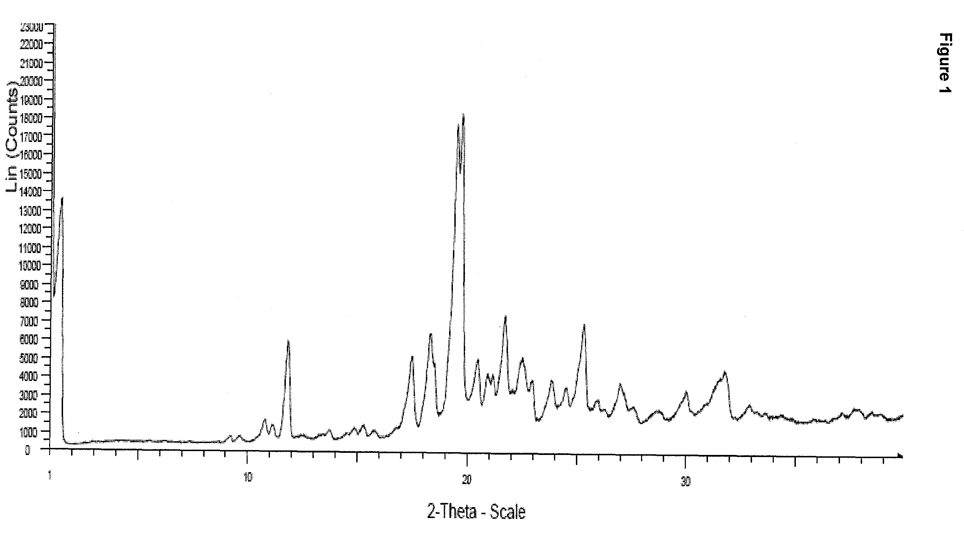

The agomelatine obtained by the process of the present invention is the polymorph form I of agomelatine which is known from Acta Cryst. (1994), C50, 907-910. Regarding the details and the definition of the crystal form, it is referred to this document. The polymorphic form I of agomelatine can also be characterized by its powder diffraction diagram which is included as figure 1. The XRPD of figure 1 is obtained from the experimental product of example 1, however, it corresponds to the theoretical XRPD that can be calculated on the basis of the full crystallographic analysis disclosed in Acta Cryst. (1994), C50, 907-910. The following table provides the relevant data of the powder diffractogram:

| Caption | Angle 2-Theta ° | d value Angstrom | Intensity % |
|---|---|---|---|
| d=9,49230 | 9,309 | 9,4923 | 4 |
| d=9,08797 | 9,724 | 9,08797 | 4,2 |
| d=8,12987 | 10,874 | 8,12987 | 9,1 |
| d=7,87082 | 11,233 | 7,87082 | 7,5 |
| d=7,41804 | 11,921 | 7,41804 | 32,3 |
| d=7,01027 | 12,617 | 7,01027 | 4,5 |
| d=6,61238 | 13,38 | 6,61238 | 4,6 |
| d=6,39308 | 13,841 | 6,39308 | 6 |
| d=6,04111 | 14,651 | 6,04111 | 5,1 |
| d=5,90901 | 14,981 | 5,90901 | 6,8 |
| d=5,75278 | 15,39 | 5,75278 | 7,7 |
| d=5,57644 | 15,88 | 5,57644 | 6 |
| d=5,22895 | 16,943 | 5,22895 | 6,9 |
| d=5,04114 | 17,579 | 5,04114 | 28 |
| d=4,80998 | 18,431 | 4,80998 | 34,8 |
| | | | |
| d=4,52600 | 19,598 | 4,526 | 96,8 |
| d=4,47643 | 19,817 | 4,47643 | 100 |
| d=4,30962 | 20,593 | 4,30962 | 27,4 |
| d=4,21408 | 21,065 | 4,21408 | 23,2 |
| d=4,16989 | 21,291 | 4,16989 | 22,9 |
| d=4,06694 | 21,836 | 4,06694 | 40,2 |
| d=3,92547 | 22,633 | 3,92547 | 27,9 |
| | | | |
| d=3,84991 | 23,084 | 3,84991 | 21,2 |
| d=3,70459 | 24,002 | 3,70459 | 21,4 |
| d=3,60834 | 24,652 | 3,60834 | 19,2 |
| | | | |
| d=3,49697 | 25,451 | 3,49697 | 38,1 |
| | | | |
| d=3,41186 | 26,096 | 3,41186 | 15,5 |
| d=3,37107 | 26,418 | 3,37107 | 12,9 |
| d=3,28161 | 27,152 | 3,28161 | 20,6 |
| | | | |
| d=3,21335 | 27,74 | 3,21335 | 13,9 |
| d=3,08937 | 28,877 | 3,08937 | 12,5 |
| d=2,96077 | 30,16 | 2,96077 | 18,5 |
| | | | |
| d=2,82576 | 31,638 | 2,82576 | 21,4 |
| d=2,80061 | 31,93 | 2,80061 | 24,6 |
| d=2,70789 | 33,054 | 2,70789 | 14,6 |
| d=2,68008 | 33,407 | 2,68008 | 12,7 |
| d=2,65048 | 33,791 | 2,65048 | 12 |
| | | | |
| d=2,59451 | 34,543 | 2,59451 | 11,9 |
| | | | |
| d=2,49011 | 36,039 | 2,49011 | 10,6 |

The polymorphic form I of agomelatine is thus also defined by the above X-ray powder diffractogram, in particular by the 2-Theta values of the peaks having an intensity of at least 30%, more preferred of the peaks having an intensity of at least 20%, most preferred of the complete diffractogram.

For the process of the present invention it is important that the agomelatine is dissolved in a water-miscible solvent to obtain a solution, and the solution is then added to water. In the prior art processes are disclosed in which agomelatine was dissolved in a water-miscible solvent, and then water was added to the solution. However, these processes did not produce agomelatine of form I but agomelatine of form II (US 2005/0182276, example 1). Furthermore, it is important that the solution with the dissolved agomelatine is added to water having a low temperature of not more than 30°C, preferably not more than 20°C, most preferred not more than 10°C. In a particularly preferred embodiment the solution of the agomelatine in the water-miscible solvent is added to water with a temperature of 0°C to 2°C, e.g. ice water, i.e. a mixture of ice and water.

If the solution of agomelatine in a water-miscible solvent is added to water having a higher temperature, mixtures of different polymorphic forms of agomelatine can be obtained, as is shown in the examples below.

The agomelatine that is used in the process of the present invention for preparing the polymorph I of agomelatine can have been prepared by any method known in the art, and it can be referred e.g. to the basic patent EP 0 447 285 or any of the other patents mentioned above which disclose processes for preparing agomelatine. Particularly preferred is that the agomelatine is prepared following the process disclosed in Yous et al. as mentioned above, but not in the biphasic (H₂O-CHCl₃) medium but in a solvent that is miscible with water. Of course, the process of Yous et al. can be strictly followed, and then the process of the present invention is carried out in order to remove the hazardous chloroform. Similarly, any known process for preparing agomelatine can be used for preparing the polymorph form I of agomelatine according to the present invention by either selecting an appropriate water-miscible solvent for the last reaction step (if the obtained product already has the required purity) or by carrying out the process of the present invention as an additional reaction step.

In the process of the present invention in a first step agomelatine is dissolved in a water-miscible solvent. The water-miscible solvent is not particularly restricted, but it is preferred that the water-miscible solvent is pharmaceutically acceptable and in particular is a solvent cited as class III solvent in the EMEA guidelines as defined above. The solvent can be a protic or an aprotic solvent. Suitable protic solvents comprise primary, secondary and tertiary alcohols with a straight or branched, cyclic or acyclic alkyl chain with one to six carbon atoms, carbon acids, carbon acid amides, primary, secondary and tertiary amines with a straight or branched, cyclic or acyclic alkyl chain with one to six carbon atoms. Preferred protic solvents comprise methanol, ethanol, propan-2-ol, acetic acid and propylamine. Methanol is not a class III solvent but a class II solvent according to the EMEA guidelines as defined above, however, the concentration limit for methanol specified by the EMEA guidelines is very high, namely 3000 ppm, and this limit can easily be achieved.

In one embodiment of the invention the water-miscible solvent is an aprotic solvent, and suitable aprotic solvents comprise aldehydes, ketones, ethers, esters, sulfoxides and carbon acid amides. Preferred aprotic solvents are acetone, 1,4-dioxane, dimethylsulfoxide and N-methylpyrrolidone. N-methylpyrrolidone like methanol is a class II solvent and not a class III solvent according to the EMEA guidelines defined above, but the concentration limit for N-methylpyrrolidone specified by the EMEA guidelines is 4840 ppm and thus even higher than the concentration limit for methanol.

Particularly preferred are methanol, ethanol, propan-2-ol, propylamine, acetic acid, acetone, N-methylpyrrolidone, 1,4-dioxane and dimethylsulfoxide.

According to the invention it is important that the solvent in which the agomelatine is dissolved in step a) is a water-miscible solvent. As used in this specification the term "water-miscible solvent" means a solvent that is miscible with water resulting in homogeneous solutions in a broad range of solvent/water ratios from 1:100 up to 100:1 (v/v), preferably at ratios from 1:20 up to 20:1 (v/v), more preferably at ratios from 1:2 to 2:1 (v/v) at a temperature of 25°C.

According to the invention it was also unexpectedly found that a very high reproducibility of the product characteristics and a very good filtrability can be obtained by using a sufficiently high excess of water. Preferably the solvent/water ratio in process step b) of the process of the present invention is at least 1:2, more preferably at least 1:3 and most preferably at least 1:4.

The amount of water can be significantly higher than the amount of solvent for agomelatine, but for practical reasons it is generally sufficient, if the solvent/water ratio is 1:10 or less or 1:8 or less or 1:6 or less. Thus, preferred solvent/water ratios are in the range from 1:2 to 1:10, preferably 1:3 to 1:8 and most preferred from 1:4 to 1:6.

All above solvent/water ratios relate to the end of the addition of the solvent to the water, i.e. when the complete solution of the agomelatine in the water-miscible solvent has been added to the water. All ratios above are v:v ratios.

It is also preferred that the solution of the water-miscible solvent with the agomelatine is added slowly to the water, preferably dropwise. Thus, in a preferred embodiment the solution in which agomelatine is dissolved in a solvent that is miscible with water is added dropwise to an excess of water. Preferably, the water is stirred during the addition of the solution.

The temperature at which the agomelatine is dissolved in the solvent that is miscible with water is not particularly critical, but it is preferred that the solution is not at an elevated temperature, and preferably the solution is at 30°C or below and most preferred at room temperature, such as 25°C or 20°C.

The concentration of the agomelatine in the water-miscible organic solvent is not particularly critical, preferred are concentrations of at least 30% of the saturation concentration, more preferred at least 50%, still more preferred at least 70% of the saturation concentration. The upper limit of the concentration of the agomelatine in the water-miscible organic solvent is generally the saturation concentration or slightly below the saturation concentration (up to 5% or 10% below the solution concentration).

When the solution containing the agomelatine dissolved in a water-miscible solvent has been added to the water, agomelatine precipitates. It is not necessary to wait for a certain amount of time after addition of the dissolved agomelatine to the water has been completed, however, it is possible to wait for some additional time to allow the crystallization to complete, e.g. stir the solution for an additional half an hour or hour. However, this is not necessary for successfully carrying out the process of the invention.

The precipitated crystals of agomelatine are then isolated usually by filtration, but, of course, other isolation methods such as centrifugation that are known in the art can also be used. The isolated crystals are optionally and preferably dried in a conventional manner, preferably at not too high a temperature of 80°C or below, preferably 70°C or below, such as 50°C. The drying of the isolated crystals can be done under reduced pressure, as is known in the art or under atmospheric pressure. Drying at a temperature range of 40°C to 80°C is carried out for generally at least one hour, usually at least two hours, until the required dryness is achieved. Generally it is not necessary to dry for more than 24 hours. Generally, the drying time is 12 to 14 h at a temperature in the range of 45 to 50°C, optionally under reduced pressure.

The crystals of polymorph form I of agomelatine obtained as described above can then be mixed with pharmaceutically acceptable excipients and processed into a pharmaceutical composition (medicament) in a manner known per se. Preferred are tablets, and suitable formulations for tablets are disclosed e.g. in US 2005/0182276. Corresponding examples are disclosed below.

Of course, it is also possible to mix the polymorphic form I of agomelatine obtained by the above process with pharmaceutically acceptable ingredients and process it to other medicaments such as capsules, pellets, sachets, etc. The polymorphic form I of agomelatine is not particularly susceptible to change to another polymorphic form, and therefore, no particular care must be taken when preparing the pharmaceutical composition.

Preferably, the pharmaceutical compositions of the present invention are free from other polymorphic forms of agomelatine and contain only the polymorphic form I of agomelatine.

The polymorphic form I of agomelatine has never been recrystallized from water-miscible organic solvents before, and therefore, the polymorphic form I of agomelatine known in the prior art contains residues of solvents which were not miscible with water. Therefore, the polymorphic form I of agomelatine that has been prepared by the process of the present invention is structurally different (due to the amount of a specific solvent) from the polymorphic form I of agomelatine obtainable by the prior art processes and is therefore a novel product. It is, of course, preferred to reduce the amount of solvent in the polymorphic form I of agomelatine according to the invention as much as possible, however, in practice it is never possible to remove all solvent molecules. The polymorphic form I of agomelatine obtainable by the process of the present invention usually contains 10 to 500 ppm, preferably 20 to 300 ppm, more preferably 50 to 200 ppm of the water-miscible solvent, preferably of methanol or ethanol. The present application also relates to this novel product. The present invention also relates to pharmaceutical compositions that have been prepared using this novel polymorphic form I of agomelatine as defined above.

The following examples further explain the present invention, however, the examples are not restrictive.

### Examples

### Example 1: Production of agomelatine form I from ethanol

20 g of agomelatine were dissolved in 80 ml ethanol. The solution was added dropwise to 600 g of ice water. Precipitated crystals were collected by filtration and dried 12 hours at 50°C. The XRPD is shown in figure 1 and is identical to the XRPD of the known polymorph I of agomelatine.

### Example 2: Production of agomelatine form I from acetone

0.5 g of agomelatine were dissolved in 1.0 ml warm acetone. The resulting solution was added dropwise to ice water. Precipitated crystals were collected and dried 12 hours at 50°C. XRPD analysis showed identical results with polymorph I.

### Example 3: Production of agomelatine form I from 2-propanol

0.5 g of agomelatine were dissolved in 20 ml propan-2-ol. The resulting solution was added dropwise to ice water. Precipitated crystals were collected and dried 12 hours at 50°C. XRPD analysis showed identical results with polymorph I.

### Example 4: Production of agomelatine form I from acetic acid

0.5 g of agomelatine were dissolved in 20 ml acetic acid. The resulting solution was added dropwise to ice water. Precipitated crystals were collected and dried 12 hours at 50°C. XRPD analysis showed identical results with polymorph I.

### Example 5: Production of agomelatine form I from propylamine

0.5 g of agomelatine were dissolved in 20 ml propan-1-amine. The resulting solution was added dropwise to ice water. Precipitated crystals were collected and dried 12 hours at 50°C. XRPD analysis showed identical results with polymorph I.

### Example 6: Production of agomelatine form I from dimethylsulfoxide

0.5 g of agomelatine were dissolved in 20 ml dimethylsulfoxide. The resulting solution was added dropwise to ice water. Precipitated crystals were collected and dried 12 hours at 50°C. XRPD analysis showed identical results with polymorph I.

### Example 7: Production of Agomelatine form I from N-methylpyrrolidone

0,5 g of Agomelatine were dissolved in 30 ml N-methylpyrrolidone. The resulting solution was added dropwise to ice water. Precipitated crystals were collected and dried 12 hours at 50 °C. XRPD analysis showed identical results with polymorph I.

### Example 8: Production of Agomelatine form I from 1,4-dioxane

0,5 g of Agomelatine were dissolved in 20 ml 1,4-dioxane. The resulting solution was added dropwise to ice water. Precipitated crystals were collected and dried 12 hours at 50 °C. XRPD analysis showed identical results with polymorph I.

### Example 9 (comparative): Production of agomelatine form II from ethyl acetate

0.5 g of agomelatine were dissolved in warm ethyl acetate and added dropwise to ice water. Precipitated crystals were collected and dried 12 hours at 50°C. XRPD analysis showed identical results with form II. Ethyl acetate is not miscible with water.

### Example 10 (comparative): Production of agomelatine form I and form II from ethanol

0.5 g of agomelatine were dissolved in 5 ml ethanol and added dropwise to water with a temperature of 40°C. Precipitated crystals were collected and dried 12 hours at 50°C. XRPD analysis showed signals characteristic for a mixture of form I and form II.

### Example 11: Production of agomelatine form I from ethanol

0.5 g of agomelatine were dissolved in 10 ml ethanol and added dropwise to water with a temperature of 20°C. Precipitated crystals were collected and dried 12 hours at 50°C. XRPD analysis showed identical results with polymorph I.

### Example 12: Pharmaceutical composition

| | |
|---|---|
| Agomelatine form I | 25 mg |
| Lactose monohydrate | 123 mg |
| Mg-Stearate | 2 mg |

The above components were mixed and filled into a capsule to provide a capsule containing a dose of 25 mg of agomelatine form I.

### Example 13: Pharmaceutical composition

| | |
|---|---|
| Agomelatine form I | 25 g |
| Lactose monohydrate | 62 g |
| Mg-Stearate | 1.3 g |
| Maize starch | 26 g |
| Maltodextrins | 9 g |
| Silica colloidal anhydrous | 0.3 g |
| Sodium starch glycolate type A | 4 g |
| Stearic acid | 2.6 g |

The above components were thoroughly mixed and pressed to 1000 tablets, each containing a dose of 25 mg in a standard tabletting machine.

## Claims

1. Process for the preparation of the polymorph from I of agomelatine, **characterized by** the following crystal data:
C₁₅H₁₇NO₂
Mr r = 243. 30
Orthorhombic
Pca2₁
a = 31.501 (4) A
b = 9.5280 (10) A
c = 17.906 (2) A
Z= 16
the process comprising the process steps of:
a) dissolving agomelatine in a water-miscible solvent to obtain a solution,
b) adding the solution to water having a temperature of 30°C or below, whereby crystals of the polymorph form I of agomelatine are formed,
c) isolating the crystals of the polymorph form I of agomelatine and
d) optionally drying the isolated crystals of the polymorph form I of agomelatine.

2. Process for the preparation of the polymorph form I of agomelatine according to claim 1, wherein in step b) the water has a temperature of 20°C or below.

3. Process for the preparation of the polymorph form I of agomelatine according to claim 2, wherein the water is ice water.

4. Process for the preparation of the polymorph form I of agomelatine according to any of claims 1 to 3, wherein the water-miscible solvent in step a) is selected from the group consisting of methanol, ethanol, propan-2-ol, acetic acid, propylamine, acetone, 1,4-dioxan, dimethylsulfoxide and N-methylpyrrolidone.

5. Process for the preparation of the polymorph form I of agomelatine according to any of claims 1 to 4, wherein step b) is carried out by dropwise addition of the solution to an excess of water.

6. Process for the preparation of the polymorph form I of agomelatine according to any of claims 1 to 5, wherein the solvent/water ratio at the end of the addition of the solution to water in step b) is at least 1:3.

7. Process for the preparation of a medicament containing as active ingredient the polymorph form I of agomelatine as defined in claim 1, comprising the following steps:
i) carrying out a process as defined in any of claims 1 to 6 in order to obtain the polymorph form I of agomelatine as defined in claim 1 and
ii) mixing the polymorph form I of agomelatine obtained in step i) with one or more pharmaceutically acceptable excipients.

8. Polymorph form I of agomelatine obtainable according to the process of any of claims 1 to 6.

9. Pharmaceutical composition comprising as active ingredient the polymorph form I of agomelatine as defined in claim 8.
